(19) Europäisches Patentamt
European Patent Office
Office européen des brevets

(11) EP 0 972 764 A1

(12) **EUROPEAN PATENT APPLICATION**

(43) Date of publication:
**19.01.2000 Bulletin 2000/03**

(21) Application number: **99113902.3**

(22) Date of filing: **16.07.1999**

(51) Int. Cl.[7]: **C07D 209/48**, C07D 209/44, C07D 211/88, C07D 211/14, C07D 207/404, C07D 207/04, C07C 215/30, C07C 225/16

(84) Designated Contracting States:
**AT BE CH CY DE DK ES FI FR GB GR IE IT LI LU MC NL PT SE**
Designated Extension States:
**AL LT LV MK RO SI**

(30) Priority: **17.07.1998 IT FI980171**

(71) Applicant:
**Universita Degli Studi di Firenze**
**50121 Florence (IT)**

(72) Inventors:
- **Guarna, Antonio**
  **50040 Seano (Carmignano) Prato (IT)**
- **Pupi, Alberto**
  **50129 Florence (IT)**
- **Berti, Giovanna**
  **54033 Carrara (Massa Carrara) (IT)**
- **Bottoncetti, Anna**
  **50011 Antella (Florence) (IT)**
- **Menchi, Gloria**
  **50019 Sesto Fiorentino (Florence) (IT)**

(74) Representative:
**Gervasi, Gemma, Dr. et al**
**NOTARBARTOLO & GERVASI**
**Corso di Porta Vittoria, 9**
**20122 Milano (IT)**

(54) **Aminoalcohols and aminoketones as CNS active agents**

(57) The present application relates to aminoalcohols and ketoamines of general formula I and to pharmaceutically acceptable salts and esters thereof, which interact with CNS receptors and can be used as drugs for the treatment of CNS diseases or, if opportunely labelled, with radioactive isotopes, as radioligands or tracers to study CNS receptors *in vitro* and *in vivo*.

EP 0 972 764 A1

## Description

### Field of the invention

The present invention relates to aminoalcohols and ketoamines of general formula I:

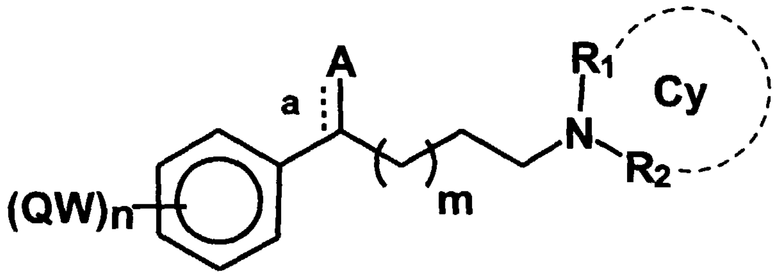

where:

$R_1$ and $R_2$, which can be equal or different from each other, are independently selected from the group consisting of H, $C_{1-8}$alkyl, $C_{2-8}$alkenyl, $C_{2-8}$alkynyl, aryl, $C_{1-8}$alkylaryl, or are linked togethere to form an imide or amide cyclic system (Cy) selected from the group consisting of:

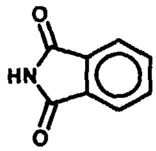 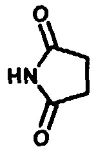 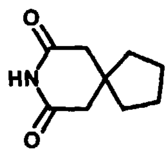 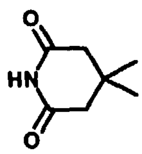

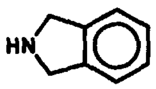 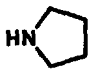 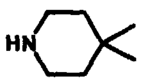 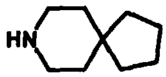

A is selected from the group consisting of O, OR, $NR_3R_4$ where R, $R_3$ and $R_4$, which can be equal or different from each other, are independently selected from the group consisting of H, $C_{1-8}$alkyl, $C_{2-8}$alkenyl, $C_{2-8}$alkynyl, aryl, aryl$C_{1-8}$ alkyl and $C_{1-8}$alkylaryl;
Q is selected in the group consisting of: simple bond, $C_{1-8}$alkyl, $C_{2-8}$alkenyl, $C_{2-8}$alkynyl, cycloalkyl, CO, CONR and NR, where R is defined as above;
W is selected from the group consisting of: H, $C_{1-8}$alkyl, $C_{2-8}$alkenyl, $C_{2-8}$alkynyl, $C_{1-8}$alkylaryl, trifluomethyl, $C_{1-8}$alkoxy, $C_{1-8}$ alkoxy-$C_{1-8}$alkyl, aryl$C_{1-8}$alkyl, aryl, aryloxyl, arylamine, $C_{1-8}$alkylcarbonyl, arylcarbonyl, arylcarboxyl, arylcarboxyamide, halogen, CN, $NR_3R_4$, $C_{1-8}$alkylamine, and a heterocycle where the groups alkyl, alkenyl, alkynyl, cycloalkyl, aryl and heterocycle can be substituted;
*n* is an integer varying between 1 and 4;
*m* is zero or 1;
the symbol ----- means that the corresponding bond 'a' can be simple or double; and their pharmaceutically acceptable salts and esters

With the provision that:

- at least one substituent group W is always an halogen atom:
- when A is OR and R is aryl, $R_1$ and $R_2$ cannot be H and $C_1$-$C_4$alkyl.
- when *m* is 1, Q is simple bond and *n* is equal to 1 or 2, W cannot be phenyl.

The invention refers also to the preparation of the above said compounds and to their use as drugs or as radioligands or tracers for the CNS.

**State of the art**

Pharmacological research is making efforts to synthesise new molecules that permit the study of the CNS and of mental diseases like Parkinson's, schizophrenia, depression, and that can be used like active compounds in drug therapy or like tracers for *in vitro* or *in viva* studies. Many drugs for the therapy of mental diseases has been commercialised in the last 30 years. However, at the moment the drugs used to treat mental diseases are successful only in 70% of patients and after a long time therapy, only the 50% of patients treated for the more common mental diseases have a complete remission of the symptoms. Indeed psychopharmacologic research is looking for antidepressive drugs with more efficacy and rapidity of action and also with very low level of toxicity and/or side effects.

Different radioligands for the binding sites of the neurotransmitters are currently used to study several mental pathologies and the mechanism of action of active drugs. Radioligands or tracers have to contain in their structure one or more atoms substituted with the correspondent radioactive isotopes, preferably they have to act at the receptors binding site as antagonistic molecules (their binding to the receptor don't elicit a biological effect) and they have to be able to cross readily the blood brain barrier (BBB).

So it is evident the importance to make available new molecules with a good affinity for CNS specific receptor population with a high rate of extraction from the BBB and that can be easily labelled with radioactive isotopes to be used as tracers to study mental illness and to monitor the stage of illness and drug therapy.

**Detailed description of the invention**

The present invention allow to answer to above-mentioned requirements and relates to the novel compounds of general formula I (as previously defined) able to interact *in vitro* and *in vivo* with CNS receptors. These compounds can be useful for the treatment of psychiatric disorders. Moreover, given that the novel compounds can be easily labelled with radioisotopes they can be used for *in vivo* and *in vitro* binding studies.

According to the present invention, $C_{1-8}$alkyl, $C_{2-8}$ alkenyl and $C_{2-8}$alkynyl are intended to encompass straight chained or branched alkyl radicals such as, for example: methyl, ethyl, propyl, isopropyl, butyl, amyl, hexyl, heptyl, octyl, ethylene, propylene, butene, isobutene, acetylene, propyne, butyne etc.

With the term cycloalkyl are indicated: cyclopropane, cyclobutane, cyclopentane, cyclohexane, cycloheptane, cyclooctane, norbornane, camphane and adamantane.

With the terms aryl are indicated: phenyl and naphthyl.

Heterocycle is used to encompass saturated or aromatic ring structures containing one or more nitrogen atoms as for example: pyridine, imidazole, pyrrole, indole, triazole, pyrrolidine, piperidine and piperazine.

Halogen means: fluorine, chlorine, bromine, iodine and their radioisotopes.

The substituents of W are preferentially selected in the group consisting of: halogen atoms, OR, phenyl, $NR_3R_4$, CN, COOR, $CONR_3R_4$ and $C_{1-8}$alkyl (where R, $R_3$ and $R_4$ are defined as above).

In particular, preferred compounds according to the present invention are those compounds of formula I where:

A = O, OR, $NR_3R_4$ where R, $R_3$ and $R_4$ are defined as above;
Q = single bond, CO, CONR, NR (where R is defined as above);
W = H, F, Cl, Br, I,$^{123}$I,$^{125}$I, $^{131}$I, $^{18}$F, methyl, t-butyl, $C_{1-8}$alkoxy, trifluoromethyl, 2,5-(di-trifluoromethyl)-phenyl, 4-metoxy-phenyl, 4-fluoro-phenyl, phenyl-$C_{1-8}$alkyl, $C_{1}$-. $_{8}$alkylcarbonyl, phenylcarbonyl, phenoxy;
n = 1 or 2 (provided that at least one of the W substituents is an halogen atom);
m = 0 or 1;
$R_1$ and $R_2$, which can be equal or different from each other, are: H, $C_{1-8}$alkyl, aryl, $C_{1-8}$alkylaryl, or, they linked together to form an imide or amide ring structure, as defined above.

Among the pharmaceutically acceptable esters and salts are included: hydrochlorides, sulfates, citrates, acetates, formates and phosphates.

According to the present invention particularly preferred compounds are:

2-[3-(4-fluorophenyl)-3-oxopropyl]-1,3-dioxo-2*H* -isoindole
2-[3-(4-fluorophenyl)-3-oxopropyl]-tetrahydro-2*H* -isoindole
1-[3-(4-fluorophenil)-3-oxopropyl]-4,4-dimethyl-2,6-dioxo-piperidine
1-[3-(4-fluorophenyl)-3-oxopropyl]-4,4-dimethyl-piperidine
8-[3-(4-fluorophenyl)-3-oxopropyl]-8-azaspiro-7,9-dioxo-[4,5]-decane

8-[3-(4-fluorophenyl)-3-oxopropyl]-8-azaspiro-[4,5]-decane
1-[3-(4-fluorophenyl)-3-oxopropyl]-2,5-dioxo-pyrrolidine
1-[3-(4-fluorophenyl)-3-oxopropyl]-pyrrolidine
*N* -benzyl-3-(4-fluorophenyl)-3-oxopropylamine
*N,N*-dibenzyl-3-(4-fluorophenyl)-3-oxopropylamine
2-[3-(4-fluorophenyl)-3-hydroxypropyl]-1,3-dioxo-2*H* -isoindole
2-[3-(4-fluorophenyl)-3-hydroxypropyl]-tetrahidro-2*H* -isoindole
1-[3-(4-fluorophenyl)-3-hydroxypropyl]-4,4-dimethyl-2,6-dioxo-piperidine
1-[3-(4-fluorophenyl)-3-hydroxypropyl]-4,4-dimethyl-piperidine
8-[3-(4-fluorophenyl)-3-hydroxypropyl]-8-azaspiro-7,9-dioxo-[4,5]-decane
8-[3-(4-fluorophenyl)-3-hydroxypropyl]-8-azaspiro-[4,5]-decane
1-[3-(4-fluorophenyl)-3-hydroxypropyl]-2,5-dioxo-pyrrolidine
1-[3-(4-fluorophenyl)-3-hydroxypropyl]-pyrrolidine
3-(4-fluorophenyl)-3-hydroxypropylamine
*N*-benzyl-3-(4-fluorophenyl)-3-hydroxypropylamine
*N,N*-dibenzyl 3-(4-fluorophenyl)-3-hydroxypropylamine
2-[3-(2-bromo-4-fluorophenyl)-3-oxopropyl]-1,3-dioxo-2*H*-isoindole
2-[3-(2-bromo-4-fluorophenyl)-3-oxopropyl]-tetrahidro-2*H*-isoindole
1-[3-(2-bromo-4-fluorophenyl)-3-oxopropyl]-4,4-dimethyl-2,6-dioxo-piperidine
1-[3-(2-bromo-4-fluorophenyl)-3-oxopropyl]-4,4-dimethyl-piperidine
8-[3-(2-bromo-4-fluorophenyl)-3-oxopropyl]-8-azaspiro-7,9-dioxo-[4,5]-decane
8-[3-(2-bromo-4-fluorophenyl)-3-oxopropyl]-8-azaspiro-[4,5]-decane
1-[3-(2-bromo-4-fluorophenyl)-3-oxopropyl]-2,5-dioxo-pyrrolidine
1-[3-(2-bromo-4-fluorophenyl)-3-oxopropyl]-pyrrolidine
*N*-benzyl-3-(2-bromo-4-fluorophenyl)-3-oxopropylamine
*N*,*N*-dibenzyl-3-(2-bromo-4-fluorophenyl)-3-oxopropylamine
2-[3-(2-bromo-4-fluorophenyl)-3-hydroxypropyl]-1,3-dioxo-2*H*-isoindole
2-[3-(2-bromo-4-fluorophenyl)-3-hydroxypropyl]-tetrahidro-2*H*-isoindole
1-[3-(2-bromo-4-fluorophenyl)-3-hydroxypropyl]-4,4-dimethyl-2,6-dioxo-piperidine
1-[3-(2-bromo-4-fluorophenyl)-3-hydroxypropyl]-4,4-dimethyl-piperidine
8-[3-(2-bromo-4-fluorophenyl)-3-hydroxypropyl]-8-azaspiro-7,9-dioxo-[4,5]-decane
8-[3-(2-bromo-4-fluorophenyl)-3-hydroxypropyl]-8-azaspiro-[4,5]-decane
1-[3-(2-bromo-4-fluorophenyl)-3-hydroxypropyl]-2,5-dioxo-pyrrolidine
1-[3-(2-bromo-4-fluorophenyl)-3-hydroxypropyl]-pyrrolidine
3-(2-bromo-4-fluorophenil)-3-hydroxypropylamine
*N*-benzyl-3-(2-bromo-4-fluorophenyl)-3-hydroxypropylamine
*N*-(4-fluorobenzyl)-3-(2-bromo-4-fluorophenyl)-3-hydroxypropylamine
*N*,*N*-dibenzyl-3-(2-bromo-4-fluorophenyl)-3-hydroxypropylamine
2-[3-(4-bromo-2-fluorophenyl)-3-oxopropyl]-1,3-dioxo-2*H*-isoindole
2-[3-(4-bromo-2-fluorophenyl)-3-oxopropyl]-tetrahydro-2*H*-isoindole
1-[3-(4-bromo-2-fluorophenyl)-3-oxopropyl]-4,4-dimethyl-2,6-dioxo-piperidine
1-[3-(4-bromo-2-fluorophenyl)-3-oxopropyl]-4,4-dimethyl-piperidine
8-[3-(4-bromo-2-fluorophenyl)-3-oxopropyl]-8-azaspiro-7,9-dioxo-[4,5]-decane
8-[3-(4-bromo-2-fluorophenyl)-3-oxopropyl]-8-azaspiro-[4,5]-decane
1-[3-(4-bromo-2-fluorophenyl)-3-oxopropyl]-2,5-dioxo-pyrrolidine
1-[3-(4-bromo-2-fluorophenyl)-3-oxopropyl]-pyrrolidine
*N*-benzyl-3-(4-bromo-2-fluorophenyl)-3-oxopropylamine
*N,N*-dibenzyl-3-(4-bromo-2-fluorophenyl)-3-oxopropylamine
2-[3-(4-bromo-2-fluorophenyl)-3-hydroxypropyl]-1,3-dioxo-2*H*-isoindole
2-[3-(4-bromo-2-fluorophenyl)-3-hydroxypropyl]-tetrahydro-2*H*-isoindole
1-[3-(4-bromo-2-fluorophenyl)-3-hydroxypropyl]-4,4-dimethyl-2,6-dioxo-piperidine
1-[3-(4-bromo-2-fluorophenyl)-3-hydroxypropyl]- 4,4-dimethyl-piperidine
8-[3-(4-bromo-2-fluorophenyl)-3-hydroxypropyl]-8-azaspiro-7,9-dioxo-[4,5]-decane
8-[3-(4-bromo-2-fluorophenyl)-3-hydroxypropyl]-8-azaspiro[4,5]-decane
1-[3-(4-bromo-2-fluorophenyl)-3-hydroxypropyl]-2,5-dioxo-pyrrolidine
1-[3-(4-bromo-2-fluorophenyl)-3-hydroxypropyl]-pyrrolidine
3-(4-bromo-2-fluorophenyl)-3-hydroxypropylamine
*N*-benzyl-3-(4-bromo-2-fluorophenyl)-3-hydroxypropylamine

*N,N*-dibenzyl-3-(4-bromo-2-fluorophenyl)-3-hydroxypropylamine
2-[3-(4-fluoro-2-iodophenyl)-3-oxopropyl]-1,3-dioxo-2*H*-isoindole
2-[3-(4-fluoro-2-iodophenyl)-3-oxopropyl]-tetrahydro-2*H*-isoindole
1-[3-(4-fluoro-2-iodophenyl)-3-oxopropyl]-4,4-dimethyl-2,6-dioxo-piperidine
1-[3-(4-fluoro-2-iodophenyl)-3-oxopropyl]-4,4-dimethyl-piperidine
8-[3-(4-fluoro-2-iodophenyl)-3-oxopropyl]-8-azaspiro-7,9-dioxo-[4,5]-decane
8-[3-(4-fluoro-2-iodophenyl)-3-oxopropyl]-8-azaspiro-[4,5]-decane
1-[3-(4-fluoro-2-iodophenyl)-3-oxopropyl]-2,5-dioxo-pyrrolidine
1-[3-(4-fluoro-2-iodophenyl)-3-oxopropyl]-pyrrolidine
*N*-benzyl-3-(4-fluoro-2-iodophenyl)-3-oxopropylamine
*N,N*-dibenzyl-3-(4-fluoro-2-iodophenyl)-3-oxopropylamine
2-[3-(4-fluoro-2-iodophenyl)-3-hydroxypropyl]-1,3-dioxo-2*H*-isoindole
2-[3-(4-fluoro-2-iodophenyl)-3-hydroxypropyl]-tetrahydro-2*H*-isoindole
1-[3-(4-fluoro-2-iodophenyl)-3-hydroxypropyl]-4,4-dimethyl-2,6-dioxo-piperidine
1-[3-(4-fluoro-2-iodophenyl)-3-hydroxypropyl]-4,4-dimethyl-piperidine
8-[3-(4-fluoro-2-iodophenyl)-3-hydroxypropyl]-8-azaspiro-7,9-dioxo-[4,5]-decane
8-[3-(4-fluoro-2-iodophenyl)-3-hydroxypropyl]-8-azaspiro-[4,5]-decane
1-[3-(4-fluoro-2-iodophenyl)-3-hydroxypropyl]-2,5-dioxo-pyrrolidine
1-[3-(4-fluoro-2-iodophenyl)-3-hydroxypropyl]-pyrrolidine
3-(4-fluoro-2-iodophenyl)-3-hydroxypropylamine
*N*-benzyl-3-(4-fluoro-2-iodophenyl)-3-hydroxypropylamine
*N,N*-dibenzyl-3-(4-fluoro-2-iodophenyl)-3-hydroxypropylamine
2-[3-(2-fluoro-4-iodophenyl)-3-oxopropyl]-1,3-dioxo-2*H*-isoindole
2-[3-(2-fluoro-4-iodophenyl)-3-oxopropyl]-tetrahydro-2*H*-isoindole
1-[3-(2-fluoro-4-iodophenyl)-3-oxopropyl]-4,4-dimethyl-2,6-dioxo-piperidine
1-[3-(2-fluoro-4-iodophenyl)-3-oxopropyl]-4,4-dimethyl-piperidine
8-[3-(2-fluoro-4-iodophenyl)-3-oxopropyl]-8-azaspiro-7,9-dioxo-[4,5]-decane
8-[3-(2-fluoro-4-iodophenyl)-3-oxopropyl]-8-azaspiro-[4,5]-decane
1-[3-(2-fluoro-4-iodophenyl)-3-oxopropyl]-2,5-dioxo-pyrrolidine
1-[3-(2-fluoro-4-iodophenyl)-3-oxopropyl]-pyrrolidine
*N*-benzyl-3-(2-fluoro-4-iodophenyl)-3-oxopropylamine
*N*,*N*-dibenzyl-3-(2-fluoro-4-iodophenyl)-3-oxopropylamine
2-[3-(2-fluoro-4-iodophenyl)-3-hydroxypropyl]-1,3-dioxo-2*H*-isoindole
2-[3-(2-fluoro-4-iodophenyl)-3-hydroxypropyl]-tetrahydro-2*H*-isoindole
1-[3-(2-fluoro-4-iodophenyl)-3-hydroxypropyl]-4,4-dimethyl-2,6-dioxo-piperidine
1-[3-(2-fluoro-4-iodophenyl)-3-hydroxypropyl]-4,4-dimethyl-piperidine
8-[3-(2-fluoro-4-iodophenyl)-3-hydroxypropyl]-8-azaspiro-7,9-dioxo-[4,5]-decane
8-[3-(2-fluoro-4-iodophenyl)-3-hydroxypropyl]-8-azaspiro-[4,5]-decane
1-[3-(2-fluoro-4-iodophenyl)-3-hydroxypropyl]-2,5-dioxo-pyrrolidine
1-[3-(2-fluoro-4-iodophenyl)-3-hydroxypropyl]-pyrrolidine
3-(2-fluoro-4-iodophenyl)-3-hydroxypropylamine
*N*-benzyl-3-(2-fluoro-4-iodophenyl)-3-hydroxypropylamine
*N,N*-dibenzyl-3-(2-fluoro-4-iodophenyl)-3-hydroxypropylamine
2-[3-(4-fluorophenyl)-3-(3-iodophenoxy)-propyl]-1,3-dioxo-2*H*-isoindole
2-[3-(4-fluorophenyl)-3-(3-iodophenoxy)-propyl]-tetrahydro-2*H*-isoindole
1-[3-(4-fluorophenyl)-3-(3-iodophenoxy)-propyl]-4,4-dimethyl-2,6-dioxo-piperidine
1-[3-(4-fluorophenyl)-3-(3-iodophenoxy)-propyl]-4,4-dimethyl-piperidine
8-[3-(4-fluorophenyl)-3-(3-iodophenoxy)-propyl]-8-azaspiro-7,9-dioxo-[4,5]-decane
8-[3-(4-fluorophenyl)-3-(3-iodophenoxy)-propyl]-8-azaspiro-[4,5]-decane
1-[3-(4-fluorophenyl)-3-(3-iodophenoxy)-propyl]-2,5-dioxo-pyrrolidine
1-[3-(4-fluorophenyl)-3-(3-iodophenoxy)-propyl]-pyrrolidine
3-(4-fluorophenyl)-3-(3-iodophenoxy)-propylamine
*N*-benzyl-3-(4-fluorophenyl)-3-(3-iodophenoxy)-propylamine
*N,N*-dibenzyl-3-(4-fluorophenyl)-3-(3-iodophenoxy)-propylamine
2-[3-(2-bromo-4-fluorophenyl)-3-(3-iodophenoxy)-propyl]-1,3-dioxo-2*H*-isoindole
2-[3-(2-bromo-4-fluorophenyl)-3-(3-iodophenoxy)-propyl]-tetrahydro-2*H*-isoindole
1-[3-(2-bromo-4-fluorophenyl)-3-(3-iodophenoxy)-propyl]-4,4-dimethyl-2,6-dioxo-piperidine
1-[3-(2-bromo-4-fluorophenyl)-3-(3-iodophenoxy)-propyl]-4,4-dimethyl-piperidine

8-[3-(2-bromo-4-fluorophenyl)-3-(3-iodophenoxy)-propyl]-8-azaspiro-7,9-dioxo-[4,5]-decane
8-[3-(2-bromo-4-fluorophenyl)-3-(3-iodophenoxy)-propyl]-8-azaspiro-[4,5]-decane
1-[3-(2-bromo-4-fluorophenyl)-3-(3-iodophenoxy)-propyl]-2,5-dioxo-pyrrolidine
1-[3-(2-bromo-4-fluorophenyl)-3-(3-iodophenoxy)-propyl]-pyrrolidine
3-(2-bromo-4-fluorophenyl)-3-(3-iodophenoxy)-propylamine
*N*-benzyl-3-(2-bromo-4-fluorophenyl)-3-(3-iodophenoxy)-propylamine
*N,N*-dibenzyl-3-(2-bromo-4-fluorophenyl)-3-(3-iodophenoxy)-propylamine
2-[3-(4-bromo-2-fluorophenyl)-3-(3-iodophenoxy)-propyl]-1,3-dioxo-2*H*-isoindole
2-[3-(4-bromo-2-fluorophenyl)-3-(3-iodophenoxy)-propyl]-tetrahydro-2*H*-isoindole
1-[3-(4-bromo-2-fluorophenyl)-3-(3-iodophenoxy)-propyl]-4,4-dimethyl-2,6-dioxo-piperidine
1-[3-(4-bromo-2-fluorophenyl)-3-(3-iodophenoxy)-propyl]-4,4-dimethyl-piperidine
8-[3-(4-bromo-2-fluorophenyl)-3-(3-iodophenoxy)-propyl]-8-azaspiro-7,9-dioxo-[4,5]-decane
8-[3-(4-bromo-2-fluorophenyl)-3-(3-iodophenoxy)-propyl]-8-azaspiro-[4,5]-decane
1-[3-(4-bromo-2-fluorophenyl)-3-(3-iodophenoxy)-propyl]-2,5-dioxo-pyrrolidine
1-[3-(4-bromo-2-fluorophenyl)-3-(3-iodophenoxy)-propyl]-pyrrolidine
3-(4-bromo-2-fluorophenyl)-3-(3-iodophenoxy)-propylamine
*N*-benzyl-3-(4-bromo-2-fluorophenyl)-3-(3-iodophenoxy)-propylamine
*N,N*-dibenzyl-3-(4-bromo-2-fluorophenyl)-3-(3-iodophenoxy)-propylamine
2-[3-(4-fluoro-2-iodophenyl)-3-(3-iodophenoxy)-propyl]-1,3-dioxo-2*H*-isoindole
2-[3-(4-fluoro-2-iodophenyl)-3-(3-iodophenoxy)-propyl]-tetrahydro-2*H*-isoindole
1-[3-(4-fluoro-2-iodophenyl)-3-(3-iodophenoxy)-propyl]-4,4-dimethyl-2,6-dioxo-piperidine
1-[3-(4-fluoro-2-iodophenyl)-3-(3-iodophenoxy)-propyl]-4,4-dimethyl-piperidine
8-[3-(4-fluoro-2-iodophenyl)-3-(3-iodophenoxy)-propyl]-8-azaspiro-7,9-dioxo-[4,5]-decane
8-[3-(4-fluoro-2-iodophenyl)-3-(3-iodophenoxy)-propyl]-8-azaspiro-[4,5]-decane
1-[3-(4-fluoro-2-iodophenyl)-3-(3-iodophenoxy)-propyl]-2,5-dioxo-pyrrolidine
1-[3-(4-fluoro-2-iodophenyl)-3-(3-iodophenoxy)-propyl]-pyrrolidine
3-(4-fluoro-2-iodophenyl)-3-(3-iodophenoxy)-propylamine
*N*-benzyl-3-(4-fluoro-2-iodophenyl)-3-(3-iodophenoxy)-propylamine
*N,N*-dibenzyl-3-(4-fluoro-2-iodophenyl)-3-(3-iodophenoxy)-propylamine
2-[3-(2-fluoro-4-iodophenyl)-3-(3-iodophenoxy)-propyl]-1,3-dioxo-2*H*-isoindole
2-[3-(2-fluoro-4-iodophenyl)-3-(3-iodophenoxy)-propyl]-tetrahydro-2*H*-isoindole
1-[3-(2-fluoro-4-iodophenyl)-3-(3-iodophenoxy)-propyl]-4,4-dimethyl-2,6-dioxo-piperidine
1-[3-(2-fluoro-4-iodophenyl)-3-(3-iodophenoxy)-propyl]-4,4-dimethyl-piperidine
8-[3-(2-fluoro-4-iodophenyl)-3-(3-iodophenoxy)-propyl]-8-azaspiro-7,9-dioxo-[4,5]-decane
8-[3-(2-fluoro-4-iodophenyl)-3-(3-iodophenoxy)-propyl]-8-azaspiro-[4,5]-decane
1-[3-(2-fluoro-4-iodophenyl)-3-(3-iodophenoxy)-propyl]-2,5-dioxo-pyrrolidine
1-[3-(2-fluoro-4-iodophenyl)-3-(3-iodophenoxy)-propyl]-pyrrolidine
3-(2-fluoro-4-iodophenyl)-3-(3-iodophenoxy)-propylamine
*N*-benzyl-3-(2-fluoro-4-iodophenyl)-3-(3-iodophenoxy)-propylamine
*N,N*-dibenzyl-3-(2-fluoro-4-iodophenyl)-3-(3-iodophenoxy)-propylamine

According to the present invention, the aminoalcohols, ketoamines and their derivatives having general formula I can be obtained following the Scheme A starting from alpha-arylchloroketones, alpha-arylchloroalcohols or alpha-arylchloroethers (2)

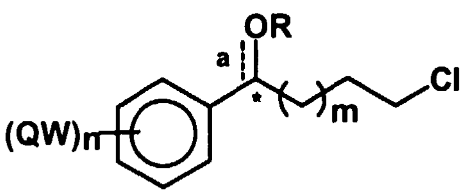

(2)

where R, Q, W, *n*, *m* and ---- are defined as above.

Compounds 2 are commercially available or they can be prepared in pure isomeric form or in racemic mixtures by known methods.

As shown in Scheme A, the preparation process of compounds I according to this invention comprises the reaction with primary or secondary cyclic or acyclic amines and with cyclic imides 3 when compounds 2 are alcohols or ethers (where a is a single bond), or only with cyclic imides when compounds 2 are ketones (where a is a double bond)

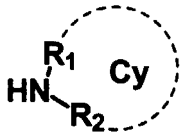

(3)

where $R_1$ and $R_2$ are defined as above and Cy represents the following cyclic amines and imides:

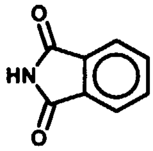 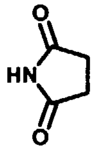 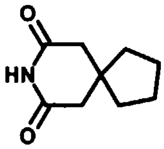 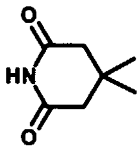

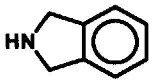 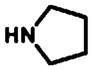 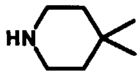 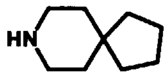

Compounds I (with A = O , a = double bond) that cannot be directly obtained from 2, can be prepared following the Scheme A by oxidation of compounds I (with A = OH, a = single bond), for example with Jones's reagent.

When compounds I are primary amine (with A = OH or OR , a = single bond and $R_1 = R_2 = H$ ), they can be transformed in the corresponding secondary amines ($R_1$ = H, $R_2$ is defined as above) for alkylation with alkyl halides ($R_2$-X) or for treatment with aldheydes ($R_2$-CHO) followed by reduction or they can be transformed into the corresponding tertiary amines ( $R_1 = R_2$ ) for alkylation with two equivalents of alkyl halides ($R_1$-X) or for treatment with two equivalents of aldheydes ($R_1$-CHO) followed by reduction. The secondary amines ($R_1$ = H, $R_2$ = alkyl) can be transformed in the corresponding tertiary amine ($R_1 \neq R_2$ are defined as above) for treatment with alkyl halides $R_1$-X

Compounds I (with A = $NHR_3$, *a* = single bond) can be prepared starting from compounds I (with A = O, *a* = double bond) by reaction with primary amines $R_3NH_2$ (where $R_3$ is defined as above), using $NaBH_3CN$ as reducing agent.

Compounds I (with A = $NR_3R_4$, *a* = single bond) can be prepared from compounds I (with A = $NHR_3$, *a* = single bond) by alkylation with an alkyl halide $R_4X$ (where $R_4$ is defined as above) in a basic medium.

Alternatively, compounds I (with A = OH or OR, *a* = single bond and Cy = cyclic amine) can be prepared by reduction with suitable reducing agents such as lithium aluminium hydride and successively basic hydrolysis starting from compounds I (with A = OH, OR *a* = single bond, Cy = cyclic imide; A = O, *a* = double bond, Cy = cyclic imide; A = OH or OR, *a* = single bond and $R_1 = R_2 = H$ ) by reaction with suitable di-halides in the presence of alkali, following known procedure.

Compounds I, prepared according to the present invention, and containing bromide, can be transformed into isotopically labelled compounds with radioactive iodine (for example $^{123}I$, $^{125}I$, $^{131}I$) by treatment with the corresponding radioactive sodium iodate, in the presence of copper I salts, following known procedures.

### Example 1

Preparation of 2-[3-(2-bromo-4-fluorophenyl)-3-hydroxypropyl]-1,3-dioxo-2*H*-isoindole [Compound I where Cy is 1,3-dioxo-2*H*-isoindole, Q = single bond, $W_n$ = 2-Br, 4-F, *n* = 2, A = OH, *m* = 0, *a* = single bond]

1.186 g (8.06 mmol) of phthalimide [compound 3 where Cy is 1,3-dioxo-2*H*-isoindole] and 1.171 g (20.15 mmol) of anhydrous potassium fluoride were added to a solution of 3-(2-bromo-4-fluorophenyl)-3-hydroxy-propylchloride [compound 2 where Q = single bond, $W_n$ = 2-bromine, 4-fluorine, *n* = 2, A = OH, *m* = 0, *a* = single bond] (1.08 g, 4.01 mmol) in 40 ml of anhydrous DMF. The reaction mixture was heated at 120 °C for 6 hours and left overnight at 25 °C.

After this time, 80 ml of $H_2O$ was added and the resulting solution extracted with diethyl ether; the organic phase was washed with $H_2O$, with a 0.5 % NaOH solution and finally with a saturated solution of NaCl and dried over anhydrous $Na_2SO_4$. After evaporation of the solvent, the solid material obtained was first washed with diethyl ether and then crystallised from acetone recovering 0.71 g (1.88 mmol) of pure compound I, in 47% yield. $^1$H-NMR ($CDCl_3$) (δ) (ppm) (Hz): 7.81 (m, 4H); 7.59 (dd, $J_1$=8.4, $J_2$=6.2, 1H); 7.20 (dd, $J_1$=8.4, $J_2$=2.6, 1H); 7.05 (td, $J_1$=8.4, $J_2$=2.6, 1H); 4.94 (d, J=11.4, 1H); 4.08-3.84 (m, 2H); 3.05 (d, J=4.4, 1H); 2.23-2.08 (m, 1H); 1.89-1.72 (m, 1H).

### Example 2

Preparation of 2-[3-(4-fluoro-2-iodophenyl)-3-(3-iodophenoxy)propyl]-1,3-dioxo-2*H*-isoindole [Compound I where Cy = 1,3-dioxo-2*H*-isoindole, Q = single bond $W_n$, = 4-F, 2-I, *n* = 2, A = 3-iodophenoxy, *m* = 0, *a* = single bond]

0.078 g (1.34 mmol) of KF and 0.07 g (0.47 mmol) of phthalimide (compound 3 where Cy è 1,3-dioxo-2*H*-isoindole) were added to 0.12 g (0.232 mmol) of 3-(4-fluoro-2-iodophenyl)-3-(3-iodophenoxy)-propyl chloride [compound 2 where Q = single bond, $W_n$ = 4-fluorine, 2-iodo, *n* = 2, A = 3-iodophenoxy, *m* = 0, *a* = single bond] dissolved in 7 ml of anhydrous DMF. The mixture was heated at 120 °C for 7 h and left overnight at 25 °C, then diluted with 10 ml of $H_2O$ and extracted with diethyl ether. After common treatment, from the organic phase were obtained 0.114 g of rough product that was purified by chromatography on silica gel recovering 0.062 g (0.099 mmol) of pure compound I in 43% yield. $^1$H-NMR ($CDCl_3$) (δ) (ppm) (Hz): 7.77 (m, 4H); 7.52 (dd, $J_1$=8.1, $J_2$=2.5, 1H); 7.28 (d, J=2.6, 1H); 7.18 (d, J=6.7, 1H); 7.00 (m, 2H): 6.85 (t, J=8.1, 1H); 6.51 (dd, $J_1$=7.7, $J_2$=2.0, 1H); 5.31 (dd, $J_1$=8.4, $J_2$=3.7, 1H); 3.98 (td, $J_1$=6.6, $J_2$=2.2, 2H); 2.16 (m, 2H).

With analogous procedures are prepared all compounds I containing cyclic imides and different substituted groups on the aromatic ring.

### Example 3

Synthesis of *N*-benzyl-3-(2-bromo-4-fluorophenyl)-3-hydroxypropylamine [Compound I with $R_1$ = H and $R_2$ = benzyl, Q = single bond $W_n$, = 2-Br, 4-F, *n* = 2, A = OH, *m* = 0, *a* = single bond]

0.075 g (1.18 mmol) of $NaBH_3CN$ in small amounts and 0.10 ml (0.104 g, 0.98 mmol, 1 eq) of benzaldehyde were added, at 0 °C, to 0.245 g (0.98 mmol) of 3-(2-bromo-4-fluorophenyl)-3-hydroxypropylamine (obtained for treatment with hydrazine, following known methods, of compounds 1 prepared as described in example 1) dissolved in 10 ml of methyl alcohol, keeping pH=6 by adding glacial acetic acid. After 24 h at 25 °C the reaction mixture was diluted with 20 ml of $H_2O$ and under agitation was added solid $Na_2CO_3$ until a basic pH was reached. After saturation of the aqueous phase with solid NaCl, the extraction was carried out with $CH_2Cl_2$. After solvent evaporation compound I was obtained (0.306 g) and purified on silica gel obtaining pure compound I as oil(78% yield).

$^1$H-NMR ($CDCl_3$) (δ) (ppm) (Hz): 7.56 (dd, $J_1$=8.4, $J_2$=6.2, 1H); 7.34 (m, 5H); 7.21 (dd, $J_1$=8.4, $J_2$=2.6, 1H); 7.01 (td, $J_1$=8.4, $J_2$=2.2, 1H); 5.19 (d, J=8.0, 1H); 3.83 (s, 2H); 2.98-2.92 (m, 2H); 2.07-1.98 (m, 1H); 1.68-1.54 (m, 1H).

### Example 4

Synthesis of *N,N*-dibenzyl-3-(2-bromo-4-fluorophenyl)-3-hydroxypropylamine [Compound I with $R_1$ = $R_2$ = Benzyl , Q = single bond, $W_n$ = 2-Br , 4-F, *n* = 2, A = OH, *m* = 0, *a* = double bond]

0.106 g of anhydrous $Na_2CO_3$ in 8 ml of anhydrous DMF and 0.066 g (0.93 mmol) of benzylbromide were added to 0.1 g (0.4 mmol) of 3-(2-bromo-4-fluorophenyl)-3-hydroxypropylamine (obtained for treatment with hydrazine, following known methods, of compounds 1 prepared as described in example 1). The mixture was heated at 70 °C for

4 h, cooled at 25 °C, diluted with 20 ml of $H_2O$ and extracted with diethyl ether. After evaporation of the organic phase and purification by chromatography on silica gel 0.120 g (0.276 mmol) of pure compound I was obtained, in 69% yield. P.f.: 82-84 °C. $^1$H-NMR ($CDCl_3$) (δ) (ppm) (Hz): 7.34 (m, 8H); 7.33-7.20 (m, 3H); 7.16 (dd, $J_1$=8.6, $J_2$=2.6, 1H); 6.87 (td, $J_1$=8.4, $J_2$=2.6, 1H); 4.94 (dd, $J_1$=8.2, $J_2$=2.4, 1H); 3.60 (dd, $J_1$=10.6, $J_2$=13.2, 4H); 2.79-2.60 (m, 2H); 1.99 (m, 1H); 1.75 (m, 1H).

**Example 5**.

Synthesis of *N,N*-dibenzyl-3-(2-bromo-4-fluorophenyl)-3-oxopropylamine [Compound I with $R_1 = R_2$ = Benzyl , Q = single bond, $W_n$ = 2-Br , 4-F, *n* = 2, A = O, *m* = 0, *a* = double bond]

0.05 ml of a 3 M solution of $Cr_2O_3$ in $H_2SO_4$ were slowly added at 0 °C to a solution of 0.05 g (0.117 mmol) of *N,N*-dibenzyl-3-(2-bromo-4-fluorophenyl)-3-hydroxypropylamine (prepared as reported in example 4) and of 0.5 ml of acetone. The mixture was left under agitation for 2 hours at 25 °C and, after solution decantation, the excess of oxidant agent was eliminated with the added of isopropyl alcohol until the solution is completely decolourised. After the added of $NaHCO_3$ until to neutral pH, the resulting solution was extracted with petroleum ether.

After usual treatment and solvent evaporation were collected 0.026 g (0.061 mmol) of solid I in 52% yield. $^1$H-NMR ($CDCl_3$)(δ) (ppm) (Hz): 7.6 (dd, $J_1$=8.4, $J_2$=2.3, 1H); 7.47-7.27 (m, 11H); 7.44 (td, $J_1$=8.1, $J_2$=2.4, 1H); 3.59 (s, 4H); 3.06 (m, 2H); 2.90-2.26 (m, 2H).

Compounds I (with A = O, *a* = double bond), prepared following example 5, can be transformed into compounds I (with A = $NHR_3$, *a* = single bond) by reaction with primary amines $R_3NH_2$ (where $R_3$ is defined as above) in presence of $NaBH_3CN$ as reducing agent by the same procedure of example 3. Compounds I (with A = $NR_3R_4$, *a* = single bond) can be prepared from compounds 1 (with A = $NHR_3$, *a* = single bond) by alkylation with an alkyl halide $R_4X$ (where $R_4$ is defined as above) following the procedure of example 4.

**Example 6**.

Preparation of 2-[3-(4-fluorophenyl)-3-hydroxypropyl]-tetrahydro-2*H*-isoindole [Compound I where Cy is tetrahydro-2*H*-isoindole, Q = single bond, $W_n$ = 4-F, *n* = 2, A = OH, *m* = 0, *a* = single bond]

0.182 g (0.612 mmol) of 2-[3-(4-fluorophenyl)-3-ossopropyl]-1,3-dioxo-2*H*-isoindole (compound I where Cy is 1 ,3-dioxo-2*H*-isoindole, Q = single bond $W_n$ = 4-fluorine, *n* = 2, A = O, *m* = 0, *a* = double bond] in 6 ml of anhydrous THF were added, very slowly at 0 °C, to a suspension of $LiAlH_4$ (0.139 g, 0.366 mmol in 8 ml of anhydrous THF. The mixture was refluxed for two hours and then, at 0 °C, 6 ml of a solution 4N of KOH and subsequently 6 ml of water were added and finally refluxed again for 30 minutes. The resulting solution, after filtration on celite, was extracted with $CH_2Cl_2$; after anhydrification and evaporation of the solvent, the residue was purified by chromatography on silica gel recovering 0.140 g of pure compound I, in 84% yield. $^1$H-NMR ($CDCl_3$) (δ) (ppm) (Hz): 7.37 (m, $J_1$=8.8, $J_2$=5.5, 2H); 7.24 (s, 4H); 7.03 (t, J=8.6, 2H); 5.00 (dd, $J_1$=7.3, $J_2$=4.3, 1H); 4.15 (dd, $J_1$=20.0, $J_2$=12.3, 4H); 3.18-3.23 (m, 2H); 2.00-1.91 (m, 2H).

**Example 7**

Synthesis of *N*-benzyl-3-(4-fluoro-2-$^{125}$I-iodo-fenil)-hydroxypropylamine [Compound I with $R_1$ = H and $R_2$ = Benzyl, Q = single bond Wn = 2 -$^{125}$I, 4-F, *n* = 2, A = OH, *m* = 0, *a* = single bond]

2.0 mg (5.91 μmol) *N*-benzyl-3-(2-bromo-4-fluorophenyl)-3-hydroxypropylamine [Compound I $R_1$ = H and $R_2$ = Benzyl, Q = single bond $W_n$ = 2-Br, 4-F, *n* = 2, A = OH, *m* = 0, *a* = single bond] were dissolved in 200 μl of ethyl alcohol and added with 642 μl of a solution x (solution x = 1.2 mg of copper (I) sulfate, 1.2 mg of tin sulfate, 30 mg of gentisic acid, 142 mg citric acid, 30 μl of glacial acetic acid, 2.7 ml deionized water) and 500 μl of ethyl alcohol. After the addition of about 2 mCi of Na$^{125}$I the solution was heated at 110 °C for 1.5 hours. After solvents evaporation, the residue was suspended in 500 μl of water and purified on Dowex AG 1X8, eluting agent: ethyl alcohol. Ethyl alcohol was eliminated by stream of nitrogen and the residue dissolved in a mixture $H_2O$/EtOH 3:7.

*In vitro* binding studies

The compounds I, prepared according to the present invention, were tested *in vitro* on preparation of rat brain membrane enriched in $D_2$ (Dopamine) or $S_2$ (Serotonine) receptors accordingly known methods to perform inhibition experiments of the binding of $^3$H-receptors ligands.

For the $D_2$ assays the method described by Ferretti et al. (Life Sci 42(24):2457-65, 1988) was used.

For the $S_2$ receptor assay the method described by List and Seeman (List S.J. and Seeman P. Proc. Natl. Acad.

Sci. USA. 78:2620-2624, 1981) was used with slight adaptations.

The data collected in the *in vitro* binding studies showed that the compound *N*-benzyl-3-(2-bromo-4-fluorophenyl)-3-hydroxypropylamine [Compound I where $R_1$ = H and $R_2$ = Benzyl, Q = single bond $W_n$ = 2-Br, 4-F, *n* = 2, A = OH, *m* = 0, *a* = single bond] which has been tested in its racemic form, has an affinity for both $S_2$ and $D_2$ receptors, displaying a dose related inhibitions of $^3$H-spiperone binding with $IC_{50}$ equal to 18.7 μM in the $S_2$ assay and 34 μM in the $D_2$ assay.

The binding affinity towards $D_2$ or $S_2$ receptors of a series of tested compounds I at 10 μM concentration are reported in Table 1.

Table 1

| **Compounds I** | $D_2$ | $T_1$ |
|---|---|---|
| 2-[3-(4-fluorophenyl)-3-oxopropyl]-tetrahydro-2*H*-isoindole | 12 | 65 |
| *N,N*-dibenzyl-3-(2-bromo-4-fluorophenyl)-3-oxopropylamine | 47 | 52 |
| *N*-benzyl-3-(2-bromo-4-fluorophenyl)-3-hydroxypropylamine | 19 | 53 |
| *N*-(4-fluorobenzyl)-3-(2-bromo-4-fluorophenyl)-3-hydroxypropylamine | 39 | 50 |
| *N*-benzyl-3-(4-bromo-2-fluorophenyl)-3-hydroxypropylamine | 26 | 60 |
| *N*,*N*-dibenzyl-3-(4-bromo-2-fluorophenyl)-3-hydroxypropyl-amine | 21 | 23 |
| *N*-benzyl-3-(4-fluoro-2-iodophenyl)-3-hydroxypropylamine | 51 | 53 |
| *N*-benzyl-3-(2-fluoro-4-iodophenyl)-3-hydroxypropylamine | 32 | 39 |
| *N*-benzyl-3-(2-bromo-4-fluorophenyl)-3-(3-iodophenoxy)-propylamine | 28 | 42 |

### *In vivo* studies

Compounds I, prepared according to the present invention and containing radioactive isotopes were tested inrespect of their capacity to cross the brain blood barrier, since CNS receptors tracers should be able to pass through the intact blood brain barrier following intravenous injection.

Measures of the rat brain extraction of the compounds was determined following known procedures based on the use of labelled reference substances with known extraction.

In the rat the first pass cerebral extraction of *N*-benzyl-3-(4-fluoro-2-$^{125}$I-iodo-fenil)-hydroxypropylamine [compound I with $R_1$ = H and $R_2$ = Benzyl, Q = single bond $W_n$ = 2-$^{125}$I, 4-F, *n* = 2, A = OH, *m* = 0, *a* = single bond] was evaluated measuring the radioactive compounds concentration in the brain, after an intracardiac injection of a mixture of the tested compound, a completely extracted tracer, as the albumin microspheres labelled with $^{99m}$Tc (undergoing to a sort of entrapment in the brain microvasculature) and a non extracted tracer as the sodium salt of hortoiodoippuric acid.

The cerebral tissue extraction fraction of *N*-benzyl-3-(4-fluoro-2-$^{125}$I-iodo-fenil)-hydroxypropylamine [Compound I with $R_1$ = H and $R_2$ = Benzyl, Q = single bond $W_n$ = 2-$^{125}$I ,4-F,*n* = 2, A = OH, *m* = 0, a = single bond] is about 50% in the rat physiological rCBF range values of 0.8-1 ml/min/g.

The experimental data suggest that the compounds of general formula I:

- have affinity for CNS dopamine and serotonine receptors in binding *in vitro* experiments
- have a cerebral tissue extraction fraction of about 50%
- can be selectively labelled by substitution of a structural bromine atom with $^{131}$I, $^{123}$I, $^{125}$I
- The non radioactive compounds can be use like neuroleptic drugs or be useful in *in vitro* experiments.
- The radioactive compounds can be use like *in vivo* tracers useful for CNS receptors imaging with PET and SPECT techniques.

Scheme A

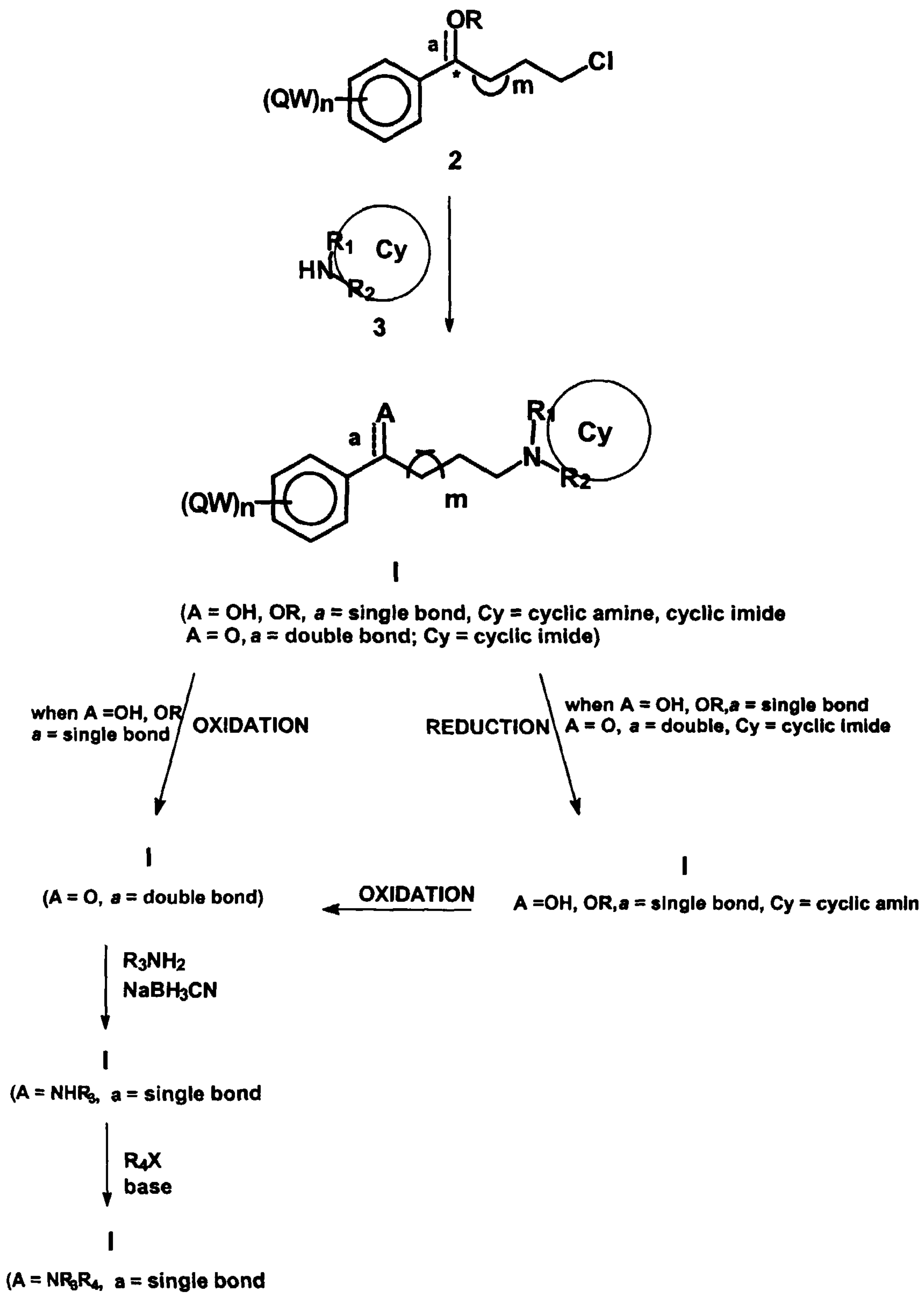
2
3
I
(A = OH, OR, a = single bond, Cy = cyclic amine, cyclic imide
A = O, a = double bond; Cy = cyclic imide)
when A =OH, OR
a = single bond
OXIDATION
REDUCTION
when A = OH, OR,a = single bond
A = O, a = double, Cy = cyclic imide
I
(A = O, a = double bond)
OXIDATION
I
A =OH, OR,a = single bond, Cy = cyclic amin
R3NH2
NaBH3CN
I
(A = NHR3, a = single bond
R4X
base
I
(A = NR3R4, a = single bond

**Claims**

**1.** Aminoalcohols and ketoamines of general formula I:

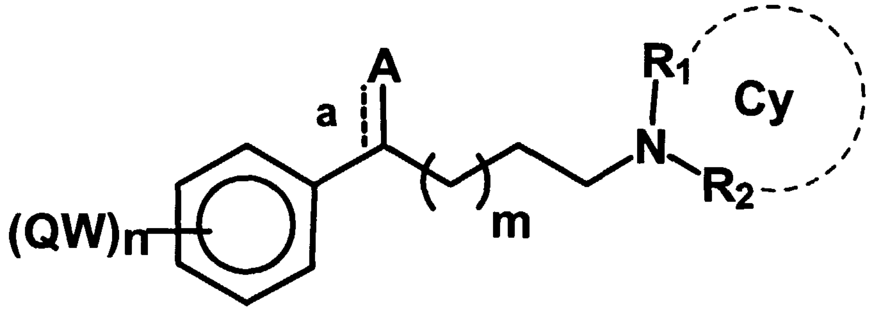

I

where:

$R_1$, $R_2$, same or different, are selected in the group consisting of H, $C_{1-8}$alkyl, $C_{2-8}$alkenyl, $C_{2-8}$alkynyl, aryl, $C_{1-8}$alkylaryl, or they are linked together to form an imide or amide cyclic system (Cy) selected from the group consisting of:

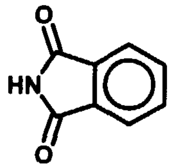 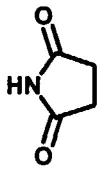 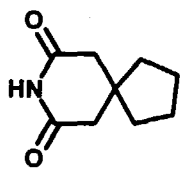 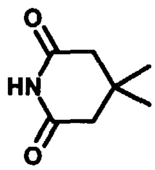

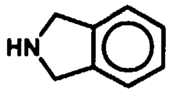 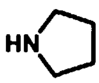 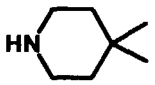 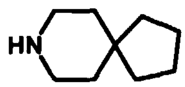

A is selected from the group consisting of O, OR and $NR_3R_4$ where R, $R_3$ and $R_4$ are independently selected in the group consisting of: H, $C_{1-8}$alkyl, $C_{2-8}$alkenyl, $C_{2-8}$alkynyl, aryl, aryl$C_{1-8}$alkyl, $C_{1-8}$alkylaryl
Q is selected in the group onsisting of single bond, $C_1$ alkyl, $C_{2-8}$ alkenyl, $C_{2-8}$alkynyl, cycloalkyl, CO, CONR and NR, where R is defined as above.
W is selected from the group consisting of: H, $C_{1-8}$alkyl, $C_{2-8}$alkenyl, $C_{2-8}$alkynyl, $C_{1-8}$alkylaryl, trifluoromethyl, $C_{1-8}$alkoxy, $C_{1-8}$ alkoxy-$C_{1-8}$alkyl, aryl$C_{1-8}$alkyl, aryl, aryloxy, arylamine, $C_{1-8}$alkylcarbonyl, arylcarbonyl, arylcarboxyl, arylcarboxyamide, halogen, CN, $NR_3R_4$, $C_{1-8}$alkylamine and heterocycle where the groups alkyl, alkenyl, alkynyl, cycloalkyl, aryl and heterocycle, can be substituted;
*n* is an integer varying between 1 e 4; *m* is zero or 1
the symbol ----- means that the corresponding bond can be single or double; provided that:

- at least one of the substituent group W is always an halogen atom:
- when A is OR and R is aryl, $R_1$ and $R_2$ cannot be H and $C_1$-$C_4$ alkyl.
- when *m* is 1, Q single bond and *n* is equal to 1 or 2, W cannot be phenyl;

and their pharmaceutically acceptable salts and esters.

**2.** Aminoalcohols and ketoamines, according to claim 1 where:

A = O, OR, $NR_3R_4$ where R, $R_3$ and $R_4$ are defined as above;

Q = single bond, CO, CONR, NR, where R is as above defined;

W = H, F, Cl, Br, I, $^{123}I$ $^{125}I$ $^{131}I$ $^{18}F$, Me, t-butyl, $C_{1-8}$alkoxy, trifluoromethyl 2,5-(di-trifluoromethyl)-phenyl, 4-methoxy-phenyl, 4-fluoro-phenyl, phenyl-$C_{1-8}$alkyl, $C_{1-8}$alkylcarbonyl, phenylcarbonyl, phenoxy;

n = 1 or 2 (provided that at least one of the substituents W be an halogen atom);

m = 0 or 1;

$R_1$ and $R_2$ are independently selected from the group consisting of: H, $C_{1-8}$alkyl, aryl and, $C_{1-8}$alkylaryl, or they are linked together to form a imide or amide cyclic system as defined above.

**3.** Aminoalcohols and ketoamines according to claim 2, represented by:

2-[3-(4-fluorophenyl)-3-oxopropyl]-1,3-dioxo-2*H*-isoindole
2-[3-(4-fluorophenyl)-3-oxopropyl]-tetrahydro-2*H*-isoindole
1-[3-(4-fluorophenyl)-3-oxopropyl]-4,4-dimethyl-2,6-dioxo-piperidine
1-[3-(4-fluorophenyl)-3-oxopropyl]-4,4-dimethyl-piperidine
8-[3-(4-fluorophenyl)-3-oxopropyl]-8-azaspiro-7,9-dioxo-[4,5]-decane
8-[3-(4-fluorophenyl)-3-oxopropyl]-8-azaspiro-[4,5]-decane
1-[3-(4-fluorophenyl)-3-oxopropyl]-2,5-dioxo-pyrrolidine
1-[3-(4-fluorophenyl)-3-oxopropyl]-pyrrolidine
*N*-benzyl-3-(4-fluorophenyl)-3-oxopropylamine
*N*,*N*-dibenzyl-3-(4-fluorophenyl)-3-oxopropyl-amine
2-[3-(4-fluorophenyl)-3-hydroxypropyl]-1,3-dioxo-2*H*-isoindole
2-[3-(4-fluorophenyl)-3-hydroxypropyl]-tetrahydro-2*H*-isoindole
1-[3-(4-fluorophenyl)-3-hydroxypropyl]-4,4-dimethyl-2,6-dioxo-piperidine
1-[3-(4-fluorophenyl)-3-hydroxypropyl]-4,4-dimethyl-piperidine
8-[3-(4-fluorophenyl)-3-hydroxypropyl]-8-azaspiro-7,9-dioxo-[4,5]-decane
8-[3-(4-fluorophenyl)-3-hydroxypropyl]-8-azaspiro-[4,5]-decane
1-[3-(4-fluorophenyl)-3-hydroxypropyl]-2,5-dioxo-pyrrolidine
1-[3-(4-fluorophenyl)-3-hydroxypropyl]-pyrrolidine
3-(4-fluorophenyl)-3-hydroxypropylamine
*N*-benzyl-3-(4-fluorophenyl)-3-hydroxypropylamine
*N*,*N*-dibenzyl 3-(4-fluorophenyl)-3-hydroxypropylamine
2-[3-(2-bromo-4-fluorophenyl)-3-oxopropyl]-1,3-dioxo-2*H*-isoindole
2-[3-(2-bromo-4-fluorophenyl)-3-oxopropyl]-tetrahydro-2*H*-isoindole
1-[3-(2-bromo-4-fluorophenyl)-3-oxopropyl]-4,4-dimethyl-2,6-dioxo-piperidine
1-[3-(2-bromo-4-fluorophenyl)-3-oxopropyl]-4,4-dimethyl-piperidine
8-[3-(2-bromo-4-fluorophenyl)-3-oxopropyl]-8-azaspiro-7,9-dioxo-[4,5]-decane
8-[3-(2-bromo-4-fluorophenyl)-3-oxopropyl]-8-azaspiro-(4,5]-decane
1-[3-(2-bromo-4-fluorophenyl)-3-oxopropyl]-2,5-dioxo-pyrrolidine
1-[3-(2-bromo-4-fluorophenyl)-3-oxopropyl]-pyrrolidine
*N*-benzyl-3-(2-bromo-4-fluorophenyl)-3-oxopropylamine
*N,N*-dibenzyl-3-(2-bromo-4-fluorophenyl)-3-oxopropylamine
2-[3-(2-bromo-4-fluorophenyl)-3-hydroxypropyl]-1,3-dioxo-2*H*-isoindole
2-[3-(2-bromo-4-fluorophenyl)-3-hydroxypropyl]-tetrahydro-2*H*-isoindole
1-[3-(2-bromo-4-fluorophenyl)-3-hydroxypropyl]-4,4-dimethyl-2,6-dioxo-piperidine
1-[3-(2-bromo-4-fluorophenyl)-3-hydroxypropyl]-4,4-dimethyl-piperidine
8-[3-(2-bromo-4-fluorophenyl)-3-hydroxypropyl]-8-azaspiro-7,9-dioxo-[4,5]-decane
8-[3-(2-bromo-4-fluorophenyl)-3-hydroxypropyl]-8-azaspiro-[4,5]-decane
1-[3-(2-bromo-4-fluorophenyl)-3-hydroxypropyl]-2,5-dioxo-pyrrolidine
1-[3-(2-bromo-4-fluorophenyl)-3-hydroxypropyl]-pyrrolidine
3-(2-bromo-4-fluorophenyl)-3-hydroxypropylamine
*N*-benzyl-3-(2-bromo-4-fluorophenyl)-3-hydroxypropylamine
*N*-(4-fluorobenzyl)-3-(2-bromo-4-fluorophenyl)-3-hydroxypropylamine
*N*,*N*-dibenzyl-3-(2-bromo-4-fluorophenyl)-3-hydroxypropylamine
2-[3-(4-bromo-2-fluorophenyl)-3-oxopropyl]-1,3-dioxo-2*H*-isoindole
2-[3-(4-bromo-2-fluorophenyl)-3-oxopropyl]-tetrahydro-2*H*-isoindole
1-[3-(4-bromo-2-fluorophenyl)-3-oxopropyl]-4,4-dimethyl-2,6-dioxo-piperidine
1-[3-(4-bromo-2-fluorophenyl)-3-oxopropyl]-4,4-dimethyl-piperidine

8-[3-(4-bromo-2-fluorophenyl)-3-oxopropyl]-8-azaspiro-7,9-dioxo-[4,5]-decane
8-[3-(4-bromo-2-fluorophenyl)-3-oxopropyl]-8-azaspiro-[4,5]-decane
1-[3-(4-bromo-2-fluorophenyl)-3-oxopropyl]-2,5-dioxo-pyrrolidine
1-[3-(4-bromo-2-fluorophenyl)-3-oxopropyl]-pyrrolidine
*N*-benzyl-3-(4-bromo-2-fluorophenyl)-3-oxopropylamine
*N*,*N*-dibenzyl-3-(4-bromo-2-fluorophenyl)-3-oxopropylamine
2-(3-(4-bromo-2-fluorophenyl)-3-hydroxypropyl]-1,3-dioxo-2*H*-isoindole
2-[3-(4-bromo-2-fluorophenyl)-3-hydroxypropyl]-tetrahydro-2*H*-isoindole
1-[3-(4-bromo-2-fluorophenyl)-3-hydroxypropyl]-4,4-dimethyl-2,6-dioxo-piperidine
1-[3-(4-bromo-2-fluorophenyl)-3-hydroxypropyl]-4,4-dimethyl-piperidine
8-[3-(4-bromo-2-fluorophenyl)-3-hydroxypropyl]-8-azaspiro-7,9-dioxo-[4,5]-decane
8-[3-(4-bromo-2-fluorophenyl)-3-hydroxypropyl]-8-azaspiro-[4,5]-decane
1-[3-(4-bromo-2-fluorophenyl)-3-hydroxypropyl]-2,5-dioxo-pyrrolidine
1-[3-(4-bromo-2-fluorophenyl)-3-hydroxypropyl]-pyrrolidine
3-(4-bromo-2-fluorophenyl)-3-hydroxypropylamine
*N*-benzyl-3-(4-bromo-2-fluorophenyl)-3-hydroxypropylamine
*N,N*-dibenzyl-3-(4-bromo-2-fluorophenyl)-3-hydroxypropyl-amine
2-[3-(4-fluoro-2-iodophenyl)-3-oxopropyl]-1,3-dioxo-2*H*-isoindole
2-[3-(4-fluoro-2-iodophenyl)-3-oxopropyl]-tetrahydro-2*H*-isoindole
1-[3-(4-fluoro-2-iodophenyl)-3-oxopropyl]-4,4-dimethyl-2,6-dioxo-piperidine
1-[3-(4-fluoro-2-iodophenyl)-3-oxopropyl]-4,4-dimethyl-piperidine
8-[3-(4-fluoro-2-iodophenyl)-3-oxopropyl]-8-azaspiro-7,9-dioxo-[4,5]-decane
8-[3-(4-fluoro-2-iodophenyl)-3-oxopropyl]-8-azaspiro-[4,5]-decane
1-[3-(4-fluoro-2-iodophenyl)-3-oxopropyl]-2,5-dioxo-pyrrolidine
1-[3-(4-fluoro-2-iodophenyl)-3-oxopropyl]-pyrrolidine
*N*-benzyl-3-(4-fluoro-2-iodophenyl)-3-oxopropyl-amine
*N*,*N*-dibenzyl-3-(4-fluoro-2-iodophenyl)-3-oxopropyl-amine
2-[3-(4-fluoro-2-iodophenyl)-3-hydroxypropyl]-1,3-dioxo-2*H*-isoindole
2-[3-(4-fluoro-2-iodophenyl)-3-hydroxypropyl]-tetrahydro-2*H*-isoindole
1-[3-(4-fluoro-2-iodophenyl)-3-hydroxypropyl]-4,4-dimethyl-2,6-dioxo-piperidine
1-[3-(4-fluoro-2-iodophenyl)-3-hydroxypropyl]-4,4-dimethyl-piperidine
8-[3-(4-fluoro-2-iodophenyl)-3-hydroxypropyl]-8-azaspiro-7,9-dioxo-[4,5]-decane
8-[3-(4-fluoro-2-iodophenyl)-3-hydroxypropyl]-8-azaspiro-[4,5]-decane
1-[3-(4-fluoro-2-iodophenyl)-3-hydroxypropyl]-2,5-dioxo-pyrrolidine
1-[3-(4-fluoro-2-iodophenyl)-3-hydroxypropyl]-pyrrolidine
3-(4-fluoro-2-iodophenyl)-3-hydroxypropylamine
*N*-benzyl-3-(4-fluoro-2-iodophenyl)-3-hydroxypropylamine
*N*,*N*-dibenzyl-3-(4-fluoro-2-iodophenyl)-3-hydroxypropylamine
2-[3-(2-fluoro-4-iodophenyl)-3-oxopropyl]-1,3-dioxo-2*H*-isoindole
2-[3-(2-fluoro-4-iodophenyl)-3-oxopropyl]-tetrahydro-2*H*-isoindole
1-[3-(2-fluoro-4-iodophenyl)-3-oxopropyl]-4,4-dimethyl-2,6-dioxo-piperidine
1-[3-(2-fluoro-4-iodophenyl)-3-oxopropyl]-4,4-dimethyl-piperidine
8-[3-(2-fluoro-4-iodophenyl)-3-oxopropyl]-8-azaspiro-7,9-dioxo-[4,5]-decane
8-[3-(2-fluoro-4-iodophenyl)-3-oxopropyl]-8-azaspiro-[4,5]-decane
1-[3-(2-fluoro-4-iodophenyl)-3-oxopropyl]-2,5-dioxo-pyrrolidine
1-[3-(2-fluoro-4-iodophenyl)-3-oxopropyl]-pyrrolidine
*N*-benzyl-3-(2-fluoro-4-iodophenyl)-3-oxopropylamine
*N*,*N*-dibenzyl-3-(2-fluoro-4-iodophenyl)-3-oxopropylamine
2-[3-(2-fluoro-4-iodophenyl)-3-hydroxypropyl]-1,3-dioxo-2*H*-isoindole
2-[3-(2-fluoro-4-iodophenyl)-3-hydroxypropyl]-tetrahydro-2*H*-isoindole
1-[3-(2-fluoro-4-iodophenyl)-3-hydroxypropyl]-4,4-dimethyl-2,6-dioxo-piperidine
1-[3-(2-fluoro-4-iodophenyl)-3-hydroxypropyl]-4,4-dimethyl-piperidine
8-[3-(2-fluoro-4-iodophenyl)-3-hydroxypropyl]-8-azaspiro-7,9-dioxo-[4,5]-decane
8-[3-(2-fluoro-4-iodophenyl)-3-hydroxypropyl]-8-azaspiro-[4,5]-decane
1-[3-(2-fluoro-4-iodophenyl)-3-hydroxypropyl]-2,5-dioxo-pyrrolidine
1-[3-(2-fluoro-4-iodophenyl)-3-hydroxypropyl]-pyrrolidine
3-(2-fluoro-4-iodophenyl)-3-hydroxypropylamine
*N*-benzyl-3-(2-fluoro-4-iodophenyl)-3-hydroxypropylamine

*N*,*N*-dibenzyl-3-(2-fluoro-4-iodophenyl)-3-hydroxypropylamine
2-[3-(4-fluorophenyl)-3-(3-iodophenoxy)-propyl]-1,3-dioxo-2*H*-isoindole
2-[3-(4-fluorophenyl)-3-(3-iodophenoxy)-propyl]-tetrahydro-2*H*-isoindole
1-[3-(4-fluorophenyl)-3-(3-iodophenoxy)-propyl]-4,4-dimethyl-2,6-dioxo-piperidine
1-[3-(4-fluorophenyl)-3-(3-iodophenoxy)-propyl]-4,4-dimethyl-piperidine
8-[3-(4-fluorophenyl)-3-(3-iodophenoxy)-propyl]-8-azaspiro-7,9-dioxo-[4,5]-decane
8-[3-(4-fluorophenyl)-3-(3-iodophenoxy)-propyl]-8-azaspiro-[4,5]-decane
1-[3-(4-fluorophenyl)-3-(3-iodophenoxy)-propyl]-2,5-dioxo-pyrrolidine
1-[3-(4-fluorophenyl)-3-(3-iodophenoxy)-propyl]-pyrrolidine
3-(4-fluorophenyl)-3-(3-iodophenoxy)-propylamine
*N*-benzyl-3-(4-fluorophenyl)-3-(3-iodophenoxy)-propylamine
*N,N*-dibenzyl-3-(4-fluorophenyl)-3-(3-iodophenoxy)-propylamine
2-[3-(2-bromo-4-fluorophenyl)-3-(3-iodophenoxy)-propyl]-1,3-dioxo-2*H*-isoindole
2-[3-(2-bromo-4-fluorophenyl)-3-(3-iodophenoxy)-propyl]-tetrahydro-2*H*-isoindole
1-[3-(2-bromo-4-fluorophenyl)-3-(3-iodophenoxy)-propyl]-4,4-dimethyl-2,6-dioxo-piperidine
1-[3-(2-bromo-4-fluorophenyl)-3-(3-iodophenoxy)-propyl]-4,4-dimethyl-piperidine
8-[3-(2-bromo-4-fluorophenyl)-3-(3-iodophenoxy)-propyl]-8-azaspiro-7,9-dioxo-[4,5]-decane
8-[3-(2-bromo-4-fluorophenyl)-3-(3-iodophenoxy)-propyl]-8-azaspiro-[4,5]-decane
1-[3-(2-bromo-4-fluorophenyl)-3-(3-iodophenoxy)-propyl]-2,5-dioxo-pyrrolidine
1-[3-(2-bromo-4-fluorophenyl)-3-(3-iodophenoxy)-propyl]-pyrrolidine
3-(2-bromo-4-fluorophenyl)-3-(3-iodophenoxy)-propylamine
*N*-benzyl-3-(2-bromo-4-fluorophenyl)-3-(3-iodophenoxy)-propylamine
*N*,*N*-dibenzyl-3-(2-bromo-4-fluorophenyl)-3-(3-iodophenoxy)-propylamine
2-[3-(4-bromo-2-fluorophenyl)-3-(3-iodophenoxy)-propyl]-1,3-dioxo-2*H*-isoindole
2-[3-(4-bromo-2-fluorophenyl)-3-(3-iodophenoxy)-propyl]-tetrahydro-2*H*-isoindole
1-[3-(4-bromo-2-fluorophenyl)-3-(3-iodophenoxy)-propyl]-4,4-dimethyl-2,6-dioxo-piperidine
1-[3-(4-bromo-2-fluorophenyl)-3-(3-iodophenoxy)-propyl]-4,4-dimethyl-piperidine
8-[3-(4-bromo-2-fluorophenyl)-3-(3-iodophenoxy)-propyl]-8-azaspiro-7,9-dioxo-[4,5]-decane
8-[3-(4-bromo-2-fluorophenyl)-3-(3-iodophenoxy)-propyl]-8-azaspiro-[4,5]-decane
1-[3-(4-bromo-2-fluorophenyl)-3-(3-iodophenoxy)-propyl]-2,5-dioxo-pyrrolidine
1-[3-(4-bromo-2-fluorophenyl)-3-(3-iodophenoxy)-propyl]-pyrrolidine
3-(4-bromo-2-fluorophenyl)-3-(3-iodophenoxy)-propylamine
*N*-benzyl-3-(4-bromo-2-fluorophenyl)-3-(3-iodophenoxy)-propylamine
*N,N*-dibenzyl-3-(4-bromo-2-fluorophenyl)-3-(3-iodophenoxy)-propylamine
2-[3-(4-fluoro-2-iodophenyl)-3-(3-iodophenoxy)-propyl]-1,3-dioxo-2*H*-isoindole
2-[3-(4-fluoro-2-iodophenyl)-3-(3-iodophenoxy)-propyl]-tetrahydro-2*H*-isoindole
1-[3-(4-fluoro-2-iodophenyl)-3-(3-iodophenoxy)-propyl]-4,4-dimethyl-2,6-dioxo-piperidine
1-[3-(4-fluoro-2-iodophenyl)-3-(3-iodophenoxy)-propyl]-4,4-dimethyl-piperidine
8-[3-(4-fluoro-2-iodophenyl)-3-(3-iodophenoxy)-propyl]-8-azaspiro-7,9-dioxo-[4,5]-decane
8-[3-(4-fluoro-2-iodophenyl)-3-(3-iodophenoxy)-propyl]-8-azaspiro-[4,5]-decane
1-[3-(4-fluoro-2-iodophenyl)-3-(3-iodophenoxy)-propyl]-2,5-dioxo-pyrrolidine
1-[3-(4-fluoro-2-iodophenyl)-3-(3-iodophenoxy)-propyl]-pyrrolidine
3-(4-fluoro-2-iodophenyl)-3-(3-iodophenoxy)-propylamine
*N*-benzyl-3-(4-fluoro-2-iodophenyl)-3-(3-iodophenoxy)-propylamine
*N*,*N*-dibenzyl-3-(4-fluoro-2-iodophenyl)-3-(3-iodophenoxy)-propylamine
2-[3-(2-fluoro-4-iodophenyl)-3-(3-iodophenoxy)-propyl]-1,3-dioxo-2*H*-isoindole
2-[3-(2-fluoro-4-iodophenyl)-3-(3-iodophenoxy)-propyl]-tetrahydro-2*H*-isoindole
1-[3-(2-fluoro-4-iodophenyl)-3-(3-iodophenoxy)-propyl]-4,4-dimethyl-2,6-dioxo-piperidine
1-[3-(2-fluoro-4-iodophenyl)-3-(3-iodophenoxy)-propyl]-4,4-dimethyl-piperidine
8-[3-(2-fluoro-4-iodophenyl)-3-(3-iodophenoxy)-propyl]-8-azaspiro-7,9-dioxo-[4,5]-decane
8-[3-(2-fluoro-4-iodophenyl)-3-(3-iodophenoxy)-propyl]-8-azaspiro-[4,5]-decane
1-[3-(2-fluoro-4-iodophenyl)-3-(3-iodophenoxy)-propyl]-2,5-dioxo-pyrrolidine
1-[3-(2-fluoro-4-iodophenyl)-3-(3-iodophenoxy)-propyl]-pyrrolidine
3-(2-fluoro-4-iodophenyl)-3-(3-iodophenoxy)-propylamine
*N*-benzyl-3-(2-fluoro-4-iodophenyl)-3-(3-iodophenoxy)-propylamine
*N,N*-dibenzyl-3-(2-fluoro-4-iodophenyl)-3-(3-iodophenoxy)-propylamine

**4.** Pharmaceutical compositions containing as active substance a therapeutically effective of a compound of formula

(I) according to Claim 1 or mixtures thereof .

5. Use of aminoalcohols and ketoamines, according to claim 1 for the preparation of pharmaceutical compositions for the treatment of CNS diseases

6. Use according to claim 5 where said CNS diseases are: Parkinson's disease, schizophrenia and depression.

7. Use of the aminoalcohols and ketoamines according to claim 1 as radioligands and tracers to study *in vitro* and *in vivo* CNS receptor systems.

8. Method for the study in vitro of CNS receptor system wherein the aminoalcohols and ketoamines according to claim 1 are used.

European Patent Office

# PARTIAL EUROPEAN SEARCH REPORT

which under Rule 45 of the European Patent Convention shall be considered, for the purposes of subsequent proceedings, as the European search report

Application Number
EP 99 11 3902

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DD 70 307 A (H. TÖNJES ET AL.) 20 December 1969 (1969-12-20) * Compounds of formula I - III * * example 10 * --- | 1,2 | C07D209/48 C07D209/44 C07D211/88 C07D211/14 C07D207/404 C07D207/04 C07C215/30 C07C225/16 |
| X | FR 2 003 340 A (DR. KARL THOMAE G.M.B.H.) 7 November 1969 (1969-11-07) * claims 1,9,15,16 * --- | 1,2,4-6 | |
| X | DE 24 51 474 A (C M INDUSTRIES) 7 May 1975 (1975-05-07) * Compounds of formula III * --- | 1,2,4-6 | |
| X | WO 94 27965 A (SYNTHEX (U.S.A.) INC.) 8 December 1994 (1994-12-08) * claims 1,2 * --- | 1,2,4-6 | |
| | -/-- | | |

TECHNICAL FIELDS SEARCHED (Int.Cl.7)

C07D
C07C

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 13 October 1999 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 99 11 3902

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | CHEMICAL ABSTRACTS, vol. 66, no. 21, 22 May 1967 (1967-05-22) Columbus, Ohio, US; abstract no. 93934h, G. M. SIMPSON ET AL.: "A new butyrophenone in the treatment of chronic schizophrenia" page 8773; XP002118628 * abstract * & PSYCHOPHARMCOLOGICA, vol. 8, no. 3, 1965, pages 223-226, --- | 1,2,4-6 | |
| X | EP 0 399 504 A (A/S FERROSAN) 28 November 1990 (1990-11-28) * claim 1; example 2 * --- | 1,2,4-6 | TECHNICAL FIELDS SEARCHED (Int.Cl.7) |
| X | US 3 308 135 A (A. B. A. JANSEN, K. E. V. SPENCER) 7 March 1967 (1967-03-07) * examples 2-6 * --- | 1,2,4-6 | |
| X | GB 1 045 244 A (JOHN WYETH & BROTHERS LTD.) 12 October 1966 (1966-10-12) * claim 1 * --- | 1,2,4-6 | |
| X | DD 66 175 A (TÖNJES ET AL.) 5 April 1969 (1969-04-05) * Compounds of formula I * --- | 1,2,4 | |
| X | EP 0 404 197 A (KYOWA HAKKO KOGYO CO., LTD.) 27 December 1990 (1990-12-27) * example 16 * --- | 1,2 | |
| X | WO 96 40097 A (NPS PHARMACEUTICALS, INC.) 19 December 1996 (1996-12-19) * claim 1 * --- | 1,2,4-6 | |
| | -/-- | | |

EPO FORM 1503 03.82 (P04C10)

European Patent Office

# PARTIAL EUROPEAN SEARCH REPORT

Application Number
EP 99 11 3902

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | E. SCHROETTER, W. WEUFFEN: "Zur bakteriostatischen Wirksamkeit von beta-Aminoketonen" PHARMAZIE, vol. 31, no. 5, 1976, pages 314-316, XP002118614 * tables 1,2 * --- | 1,2,4 | |
| X | E. SCHRÖTTER, M. TSCHÄPE: "Anästhetisch wirksame beta-Aminoketone aus 3-Chlor-4-n-alkoxy-5-methylacetophenonen" PHARMAZIE, vol. 31, no. 1, 1976, pages 21-24, XP002118615 * tables 1-9 * --- | 1,2,4 | TECHNICAL FIELDS SEARCHED (Int.Cl.7) |
| X | G. CASCIO ET AL.: "4-Aminobutirrofenoni ad attivita ipotensiva" FARMACO ED. SCI., vol. 31, no. 6, 1976, pages 442-456, XP002118616 * table 1 * --- | 1,2,4-6 | |
| X | C. CARGANICO ET AL.: "Synthesis of some 3-hydroxy-5-aminopentanoic acid derivatives" FARMACO ED. SCI., vol. 42, no. 4, 1987, pages 277-284, XP002118617 * Scheme I * --- | 1,2,4-6 | |
| | -/-- | | |

EPO FORM 1503 03.82 (P04C10)

European Patent Office

# PARTIAL EUROPEAN SEARCH REPORT

Application Number
EP 99 11 3902

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | Y. HUANG, I. H. HALL: "Hypolipidemic Activity of 3-Amino-1-(2,3,4-mononitro-, mono-, or dihalophenyl)propan-1-ones in Rodents" ARCH. PHARM. (WEINHEIM), vol. 329, no. 7, 1996, pages 339-346, XP002118618 * tables 1-9 * --- | 1,2,4 | |
| X | A. BUSCHAUER: "Synthesis and in Vitro Pharmacology of Arpromidine and Related Phenyl(pyridylalkyl)guanidiens, a Potential New Class of Positive Inotropic Drugs" J. MED. CHEM., vol. 32, no. 8, 1989, pages 1963-1970, XP002118619 * page 1968 - page 1969 * --- | 1,2,4 | TECHNICAL FIELDS SEARCHED (Int.Cl.7) |
| Y | F. COLLINO, M. DE NARDO: "Mannich ketobases with narcotic-antagonistic activity" BOL. CHIM. FARM., vol. 122, no. 8, 1983, pages 393-404, XP002118620 * tables VII,,VIII * --- | 1-8 | |
| X | V. N. PATHAK, R. P. SINGH: "Studies in Fluorinated Bases" PHARMAZIE, vol. 35, no. 7, 1980, page 434 XP002118621 * table 1 * --- | 1,2,4 | |
| Y | US 3 983 133 A (J. L. H. VAN GELDER ET AL.) 28 September 1976 (1976-09-28) * column 7, line 1 - line 65 * --- | 1-8 | |

-/--

EPO FORM 1503 03.82 (P04C10)

European Patent Office

# PARTIAL EUROPEAN SEARCH REPORT

Application Number
EP 99 11 3902

| Category | Citation of document with indication, where appropriate, of relevant passages | Relevant to claim | CLASSIFICATION OF THE APPLICATION (Int.Cl.7) |
|---|---|---|---|
| Y | E. D. BERGMANN, Z. GOLDSCHMIDT: "Some Epinephrine Analogs" J. MED. CHEM., vol. 11, no. 6, 1968, pages 1121-1125, XP002118622 * table I * --- | 1-8 | |
| X | J. YOVELL ET AL.: "Acid-Catalyzed Addition of Secondary Amines to Cyclopropyl Ketones. Mass Spectra of Some Cyclic Aminobutyrophenones" J. ORG. CHEM., vol. 42, no. 5, 1977, pages 850-855, XP002118623 * table I * --- | 1,2 | TECHNICAL FIELDS SEARCHED (Int.Cl.7) |
| X | I. UEDA, S. UMIO: "The Synthesis of 11-(2'-Dimethylaminoethyl)-5-methyl-5,11-dihydrodibenzo[b,e][1,4]thiazepin and Related Compounds. Neurotropic and Psychotropic Agents" BULL. CHEM. SOC. JPN., vol. 48, no. 8, 1975, pages 2323-2327, XP002118624 * Compounds of formula 6, 7 and 9 * --- | 1,2 | |
| X | I. FLEMING, M. WOOLIES: "A New Synthesis of Indoles Particularly Suitable for the Synthesis of Tryptamines and Tryptamine Itself" J. CHEM. SOC. PERKIN TRANS. 1,1979, pages 829-837, XP002118625 * Compounds of formula 13 * --- | 1,2 | |
| | -/-- | | |

EPO FORM 1503 03.82 (P04C10)

European Patent Office

# PARTIAL EUROPEAN SEARCH REPORT

Application Number
EP 99 11 3902

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | S. D. SAHASRABUDHE, B. D. TILAK: "Synthesis of Heterocyclic Compounds: Part XXVIII - Potential Antilukemic Agents from 3,4-Dihydro-1,3,2-oxazaphosphorin-2-oxides " INDIAN J. CHEM. SECT. B, vol. 23, no. 10, 1984, pages 914-917, XP002118626 * Compunds of formula 3 and 5 * --- | 1,2 | |
| X | Y. HUANG, I. HALL: "Anti-inflammatory activity of alpha-, beta- and gamma-alkylaminoketones in CF(1) mice" RES. COMMUN. PHARMACOL. TOXICOL., vol. 1, no. 1, 1996, pages 17-38, XP002118627 * figure 1 * ----- | 1,2,4 | TECHNICAL FIELDS SEARCHED (Int.Cl.7) |

EPO FORM 1503 03.82 (P04C10)

European Patent Office

**INCOMPLETE SEARCH SHEET C**

Application Number
EP 99 11 3902

Claim(s) searched completely:
3

Claim(s) searched incompletely:
1-2,4-8

Reason for the limitation of the search:

The initial phase of the search revealed a very large number of documents relevant to the issue of novelty. So many documents were retrieved that it is impossible to determine which parts of the claims may be said to define subject-matter for which protection might legitimately be sought (Article 84 EPC). For these reasons, a meaningful search over the whole breadth of the claims is impossible. Consequently, the search has been restricted to:

The compounds represented by the examples and the compounds claimed in claim 3.

## ANNEX TO THE EUROPEAN SEARCH REPORT ON EUROPEAN PATENT APPLICATION NO. EP 99 11 3902

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-1999

| Patent document cited in search report | Publication date | Patent family member(s) | Publication date |
|---|---|---|---|
| DD 70307 A | | NONE | |
| FR 2003340 A | 07-11-1969 | BE 729473 A | 08-09-1969 |
| | | DK 125242 B | 22-01-1973 |
| | | NL 6903457 A | 09-09-1969 |
| | | SE 366025 B | 08-04-1974 |
| DE 2451474 A | 07-05-1975 | GB 1475314 A | 01-06-1977 |
| | | AT 337165 B | 10-06-1977 |
| | | AT 866574 A | 15-10-1976 |
| | | BE 821571 A | 17-02-1975 |
| | | CA 1045137 A | 26-12-1978 |
| | | CH 593907 A | 30-12-1977 |
| | | DD 115110 A | 12-09-1975 |
| | | DK 570374 A | 23-06-1975 |
| | | ES 431312 A | 16-10-1976 |
| | | FR 2249659 A | 30-05-1975 |
| | | JP 50077351 A | 24-06-1975 |
| | | NL 7414310 A | 07-05-1975 |
| | | US 4104383 A | 01-08-1978 |
| WO 9427965 A | 08-12-1994 | AT 171446 T | 15-10-1998 |
| | | AU 680004 B | 17-07-1997 |
| | | AU 6954894 A | 20-12-1994 |
| | | BR 9406724 A | 06-02-1996 |
| | | CA 2163747 A | 08-12-1994 |
| | | CN 1124485 A | 12-06-1996 |
| | | CZ 9502780 A | 12-06-1996 |
| | | DE 69413535 D | 29-10-1998 |
| | | DE 69413535 T | 11-02-1999 |
| | | EP 0700383 A | 13-03-1996 |
| | | ES 2121210 T | 16-11-1998 |
| | | FI 955660 A | 24-11-1995 |
| | | HU 9500112 A | 28-06-1995 |
| | | HU 74870 A | 28-02-1997 |
| | | IL 109776 A | 10-03-1998 |
| | | JP 8510743 T | 12-11-1996 |
| | | NO 954761 A | 26-01-1996 |
| | | NZ 267296 A | 24-04-1997 |
| | | PL 311723 A | 04-03-1996 |
| | | US 5763458 A | 09-06-1998 |
| EP 399504 A | 28-11-1990 | AT 122031 T | 15-05-1995 |
| | | AU 641653 B | 30-09-1993 |
| | | AU 5582190 A | 29-11-1990 |
| | | CA 2017468 A | 26-11-1990 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 99 11 3902

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-1999

| Patent document cited in search report | Publication date | Patent family member(s) | Publication date |
|---|---|---|---|
| EP 399504 A | | DE 69019060 D | 08-06-1995 |
| | | DE 69019060 T | 31-08-1995 |
| | | DK 399504 T | 04-09-1995 |
| | | ES 2071699 T | 01-07-1995 |
| | | IE 70070 B | 30-10-1996 |
| | | IL 94452 A | 27-11-1995 |
| | | JP 3017046 A | 25-01-1991 |
| | | NO 174342 B | 10-01-1994 |
| | | NZ 233809 A | 28-04-1993 |
| | | PT 94167 A,B | 08-01-1991 |
| | | US 5145870 A | 08-09-1992 |
| | | US 5310756 A | 10-05-1994 |
| US 3308135 A | 07-03-1967 | GB 1045244 A | |
| GB 1045244 A | | US 3308135 A | 07-03-1967 |
| DD 66175 A | | NONE | |
| EP 404197 A | 27-12-1990 | CA 2019621 A | 23-12-1990 |
| | | JP 3178952 A | 02-08-1991 |
| | | US 5128369 A | 07-07-1992 |
| WO 9640097 A | 19-12-1996 | AU 6112596 A | 30-12-1996 |
| | | BR 9609019 A | 06-07-1999 |
| | | CA 2223978 A | 19-12-1996 |
| | | CN 1192679 A | 09-09-1998 |
| | | EP 0831799 A | 01-04-1998 |
| | | JP 11506469 T | 08-06-1999 |
| | | PL 323871 A | 27-04-1998 |
| US 3983133 A | 28-09-1976 | US 3923808 A | 02-12-1975 |

EPO FORM P0459

For more details about this annex : see Official Journal of the European Patent Office, No. 12/82